Europäisches Patentamt

European Patent Office

Office européen des brevets

(19)

(11) **EP 0 377 549 B1**

(12) **EUROPEAN PATENT SPECIFICATION**

(45) Date of publication and mention
of the grant of the patent:
**09.12.1998 Bulletin 1998/50**

(51) Int Cl.6: **G01L 1/24**, G01D 5/26,
G01K 11/20, G06K 11/16,
G01K 11/18

(21) Application number: **90630001.7**

(22) Date of filing: **03.01.1990**

(54) **Remote measurement of physical variables with fiber optic systems**

Fernmessung physikalischer Variablen mit faseroptischen Systemen

Télédétection des variables physiques par des systèmes à fibre optique

(84) Designated Contracting States:
**BE CH DE DK FR GB IT LI LU NL SE**

(30) Priority: **03.01.1989 US 293119**

(43) Date of publication of application:
**11.07.1990 Bulletin 1990/28**

(73) Proprietor: **Kleinerman, Marcos Y.**
**Southbridge Massachusetts 01550 (US)**

(72) Inventor: **Kleinerman, Marcos Y.**
**Southbridge Massachusetts 01550 (US)**

(74) Representative: **Waxweiler, Jean et al**
**Dennemeyer & Associates Sàrl**
**P.O. Box 1502**
**1015 Luxembourg (LU)**

(56) References cited:
**EP-A- 32 990**      **EP-A- 95 673**
**DE-A- 3 247 659**      **DE-A- 3 712 699**
**GB-A- 2 156 513**      **US-A- 4 373 768**
**US-A- 4 770 492**      **US-A- 4 834 496**

**Description**

Background of the Invention

The present invention relates to improved methods and devices for the remote measurement of physical variables with fiber optic systems, and more particularly is directed to the novel use of forward time division multiplexing techniques for increasing the measuring capability of sensing systems and the accuracy of the measuremnts made with them.

Description of the Prior Art.

Fiber optic sensing systems have been under development in recent years for the remote measurement of physical variables (also referred to as measurands) of interest in industry, medicine, transportation, and other fields of human endeavor. There are two main approaches to fiber optic sensing, as follows:

(a) The use of the optical fiber itself as the transducer, in a system which may be interferometric or non-interferometric, and
(b) The use of a point transducer attached or otherwise optically connected to one or more optical fibers, the latter acting merely as light guides.

These and other approaches are discussed in a recent review by G.D. Pitt et. al., IEE Proceedings, Vol 132, Pt. J, No. 4, August 1985, pp214-248.

In addition, optical fibers are used for transmitting the information of sensors, whether optical or electrical, to a remote control station.

Interferometric sensing systems use the fibers themselves as transducers,and can achieve high sensitivities. They have, however, several disadvantages. They usually require single node lasers, single mode fibers,and relatively complex instruments, and are often subject to drift and phase noise due to environmental factors other than the parameter sought to be measured. Further, they are not presently compatible with the industrial requirements for ruggedness. Non-interferometric systems, which usually use multimode fibers and non-coherent light sources, are simpler, more rugged, and are capable of meeting most of the sensitivity requirements of industry. The methods and devices of this invention are based on non-interferometric systems.

Many fiber optic sensing systems are based on light intensity changes produced by the action of the measurand. In order to obtain a reliable measurement of the physical variable with such systems, it is necessary to compare the optical signal generated by the measurand to a reference signal which is not affected by this measurand. This is done in the prior art by any of the following ways:

(a) If the sensor is not spectrally selective, by splitting the light output from the interrogating light source into two separate beams each carried by a separate fiber. One of the beams is made to interact with the measurand, where its intensity is modulated according to the measurand's value. The other beam is used as a reference. The modulated beam and the reference beam are sent through separate optical paths to separate photodetectors, and the resulting electrical signals are compared. The value of the measurand is determined from the relative values of the two electrical signals.
(b) If the sensor is spectrally selective, by interrogating the probe with light of an intensity distributed in a known manner between two wavelength regions, the intensities of each of these spectral components modulated in a known, different manner by the probe. There are two ways to accomplish this. One way is to carry both spectral components simultaneously by the same fiber, and then to separate them at the fiber output (after interaction with the sensor) by means of wavelength-selective optical filters, feeding each separate beam to a separate photodetector. The other way is to use a switching device to send both spectral components alternately through the same fiber system to the same photodetector. The latter methol was used to measure temperature by monitoring the temperature-dependent light transmission of semiconductor crystals within their absorption band edge (Kyuma et. al., IEEE J. Quant. Electron., QE-18(4), 677 (1982)).

Each of the above methods is subject to error, due to unequal detector drift, varying losses in the separate optical paths and/or unequal fluctuations in the intensities of the spectral components of the interrogating light source(s).

In the prior art sensors described above, so-called intensity sensors, the value of the measurand is determined indirectly from the attenuation of the intensity $P_o$ of the interrogating light incident on the sensor to a transmitted value $P_o (1 - \alpha)$. The value of $\alpha$, which is an indicator of the value of the measurand, is estimated indirectly from the measurement of the transmitted light intensity. It is well known that such measurements can not be made with a high degree of accuracy when the optical density is of the order of $10^{-3}$ or lower <u>for a single, discrete sensor</u>. The accuracy of said attenuation measurements is further degraded in the case of series-multiplexed sensors, where the attenuation measurements must be carried out by optical time domain reflectometry (OTDR) techniques, which consist of the measurement of the decrease of the intensity of the Rayleigh-backscattered light caused by each sensor. In this case a measurement with an accuracy of one percent may require the accurate and reproducible measurement of light intensity changes of a few parts per $10^4$. This is difficult to achieve with a relatively simple device, especially considering that Rayleigh-backscattered light signals are reletively weak, with an intensity <u>down</u> about 45 to 50 dB with respect to the intensity of the interrogating light at the same fiber location.

Most non-interferometric fiber optic sensing systems operate by measuring the measurand-dependent variable light transmission of the sensor, and their performance is subject to the inherent limitations discussed in the preceding paragraph. Temperature-sensing systems include, besides the one already mentioned by Kyuma <u>et. al</u>., the systems subject of U.S. patents No. 4,136,566 (Christensen), 4.302,970 (Snitzer <u>et al.)</u>, 4,278,349 (Sander), 4,307,607 (Saaski <u>et. al.)</u>. and the cryogenic temperature sensor described in NASA Technical Briefs, p.55, August, 1981). <u>All</u> of these systems are based on a temperature-dependent light attenuation.

Other sensor systems based on variable light attenuation and designed to measure a variety of measurands include the ones described in U.S. patents No. 4,356,448 (Brogardh <u>et al.)</u>, 4,433,238 (Adolfsson <u>et. al. )</u>, and 4,523,092 (Nelson). There are many others, but they fall within the same category of attenuation sensors covered by these references.

Fiber optic sensing techniques are especially suited for distributed sensing and for the multiplexing of a multiplicity of discrete sensors, and considerable interest has developed in recent years in these applications. A comprehensive review has been published recently (J.P. Dakin, <u>J. Phys. E: Sci. Instrum 20</u>, 954 (1987)), which discusses many approaches suggested or under development for these purposes. Some of these methods and approaches were first disclosed after the filing of the US application (serial No. 405,732). OTDR techniques seem to be most extensively investigated, mainly coupled to the measurement of Rayleigh-backscattered light, but Raman and fluoresence variants have also been proposed. An anti-Stokes Raman technique has been proposed for measuring temperature (Dakin et. al., Electron. Lett. 21 569 (1985)) based on the temperature-dependent occupancy number of a vibrationally excited level in a glass. The method provides signals which are orders of magnitude weaker than a method, also based on a temperature-dependent occupancy number of a vibrationally excited molecular level, subject of said US application serial No. 405,732 and using fluorescence conversion, and can be regarded as a less sensitive variant of said earlier invention. A recently proposed fluorescence technique proposed by Dakin, mentioned in said review article, is based on temperature-dependent changes in the fluorescence spectral distribution of some fluorescent dyes, in contrast to the methods of this invention, which do <u>not</u> require any temperature-dependent change in any fluorescence property and can, therefore, be implemented with most dyes.

A method for measuring distributed forces has recently been proposed by Farries and Rogers and discussed in another review article (A.J. Rogers, <u>J.Phys. D: Appl. Phys. 19</u> 2237 (1986)), based on the effect of force-induced changes of the polarization properties of a single mode fiber on the stimulated Raman gain, under intense 'pumping' optical pulses, from sensing points along the optical fiber. The method has the disadvantage (among others) that polarization changes at one point along the fiber affect the polarization properties of the fiber along the rest of its length, and the results are thus difficult to interpret.

Macedo <u>et. al.</u> (U.S. patent No. 4,342,907) describe an interesting OTDR-based system for the measurement of distributed forces acting at different <u>pre-determined</u> points along a fiber optic cable. These forces divert a fraction of the intensity of the interrogating light propagating along the fiber from the fiber core into the cladding. This diverted light is removed by a coupler mechanically attached to the cable at each sensing point and transferred either to a second fiber optic cable (the return light guide) by means of another coupler, which directs this light to a photodetector in the OTDR device, or reflected back by an external detector into the same fiber optic cable towards the OTDR device. While the system has the advantage that it produces optical signals which are a linear function of the intensity of the light forced out of the core, and are free of the baseline background of the interrogating light intensity, it is mechanically and optically complex, requiring at least one directional coupler per sensing point, and is unsuitable for detecting or measuring forces at points other than pre-determined ones.

It is an object of the present invention to provide methods and devices which eliminate or minimize the sources of error discussed above, said methods and devices producing both a single beam and a reference beam from a single light source, whether this be a broad band or a narrow band or monochromatic source, carrying both beams simultaneously through a single fiber to a single photodetection station, and separating, measuring and ratioing by simple means the electrical signals generated at the photodetection station by both the signal beam and the reference beam.

It is another object of this invention to provide a simple method for measuring directly and accurately small attenuations of an interrogating light beam produced by the action of a measurand, by generating a signal light intensity proportional to said attenuation and free of the intensity of the interrogating light incident on or transmitted by the sensor.

Still another object of the present invention is to provide new methods and devices for the measurement of diverse measurands at a plurality of remote locations simultaneously or quasi-simultaneously, using a single excitation light source and a single photodetector, with the individual probes attached to a single unbroken optical fiber, said measurements being only minimally affected by fluctuations of the intensity of the interrogating light beam, fiber losses or detector drift.

Yet another object of the present invention is to provide a long unbroken optical fiber as a distributed sensor, said fiber being sensitive to both mechanical forces and temperatures acting at different points along the fiber, and associated devices for the accurate measurement of the location and magnitude of said distributed temperatures and mechanic forces.

Other objects of the present invention will in part be apparent from the following discussion and will in part appear hereinafter.

Brief Summary of the Invention

These and other objects are accomplished by using so-called intensity sensors comprising an optical fiber according to claim 1. In accordance with the invention interrogating light is launched into the core of an optical fiber probe for a measurand, wherein a fraction $\alpha$ of its initial intensity is removed from the core, the value of $\alpha$ being an indicator of the value of the measurand. Instead of discarding this removed fraction as in the prior art and estimating its value indirectly from the light intensity $P_o(1 - \alpha)$ transmitted by the probe, the removed light is captured and processed into an optical signal separable from the transmitted interrogating light and from signals from measurands acting simultaneously at any other point(s) on the fiber, but carried by the same fiber to a photodetection station, so that both the intensities of the transmitted interrogating light light beam and that of said removed fraction $\alpha$, the value of $\alpha$ being an indicator of the value of the physical parameter, can be measured to give a ratiometric (referenced) reading of the value of the measurand, unaffected or only minimally affetced by fluctuations of the intensity of the interrogating light, fiber losses or detector drift. The processing of $\alpha$ into a separable, directly measurable signal, can be effected either in the optical spectral domain, by luminescence or Raman conversion into a light of different wavelengths from those of the interrogating light, or as in the present invention in the time domain, using pulsed or AC-modulated interrogating light and processing said fraction $\alpha$ into a signal which arrives at the photodetector either at a different time or with a phase shift relative to the interrogating light. An important feature of the invention is that the sensing materials it uses can be homogeneously incorporated into components of long optical fibers, allowing the measurement of temperatures and/or forces distributed over many locations, simultaneously and with a single fiber probe.

Definitions and Symbols

Within the context of this application, I am using the following definitions:

Light: optical radiation, whether or not visible.

Measurand, or physical variable, or physical parameter: any physical property which can change in value. Examples: temperature, force, flow rate, level, position, and the like.

Interrogating Light: Light launched into the fiberoptic system and which is modulated by the physical parameters being measured.

Excitation light: interrogating light which generates a luminescence or Raman-shifted light of an intensity determined by the value of the measurand.

Force: any action which, on an optical fiber, affects the transmission of light along it. Examples: stress, pressure, sound waves, and the like.

$\lambda_s$: a wavelength of interrogating light within an absorption band of a sensing probe of this invention, used for sensing a force or forces acting on the probe.

$\lambda_f$: a wavelength or wavelengths of light generated at a sensing probe by the interrogating light, different from the wavelength(s) of the interrogating light.

$\alpha$ : the fraction of the intensity of an interrogating light attenuated within a sensing probe at a sensing point.

$\alpha_s$ and $\alpha/_v$: tractions of the intensity of an interrogating light of wavelengths $\lambda_s$ or $\lambda_v$, respectively, attenuated within a sensing probe at a sensing point.

Effective Optical Path Length of a Light Guide: the product of the actual path length of the light guide times its index of refraction.

Sensor: a probe or any other object which undergoes an observable change of a measured quantity under the effect of a physical variable.

Cladding: a light-guiding region surrounding a fiber core, whether or not it is in physical contact with the core, and regardless of the value of its index of refraction.

BRIEF DESCRIPTION OF THE DRAWINGS

FIGS 1 and 2 describe two optical fibers useful for practicing this invention, having a luminescent cladding.

FIGS 3, 3A and 3B illustrate an arrangement for the measurement of distributed forces using optical fibers having two light-guiding regions of different effective optical path length.

FIG. 4 and 4A illustrate a distributed optical thermometer according to this invention.

FIGS 5, 5A and 5B illustrate fiberoptic keyboards according to this invention.

DETAILED DESCRIPTION OF THE INVENTION

1.0- The Direct Measurement of Small Optical Attenuations Caused by Physical Variables.

In fiber optic, sensing technology, optical attenuations are conventionally measured by <u>indirect</u> techniques. As the following example shows, such approach is not suitable for the measurements of small attenuations. Suppose that one is measuring temperature with an optical probe characterized by a temperature-dependent optical absorption coefficient at a wavelenght $\lambda_v$. The optical density of the probe is low enough that, at a temperature of 300 kelvins (K), the probe absorbs a fraction $\alpha_v$ equal to 0.0100 of the power $P_o$ of the interrogating light at that wavelength. The fraction $\alpha_v$ is not measured <u>directly</u> in the prior art in optical sensors. What is measured is the power P of the light <u>transmitted by the probe.</u> This is, disregarding scattering or other losses, equal to 0.9900 $P_o$, The value of $\alpha_v$ is <u>derived</u> from the difference $(P_o-P)/P_o$. Assure now that at 300K the temperature coefficient of $\alpha_v$ is equal to 1.67 percent per K, that is, a temperature increase of 1 K increases $\alpha_v$ by 1.67 percent. But one measures P, <u>not</u> $\alpha_v$, and when $\alpha_v$ changes by 1.67 percent P changes by only 0.017 percent, a change of <u>less than 2 parts per 10,000</u>. Such a small change is difficult to measure accurately with ordinary photometric equipment.

Using the principles of this invention, one can measure $\alpha_v$ directly, and obtain an actual, directly measurable light intensity which <u>does</u> vary by 1.67 percent per kelvin. To do this, one uses a probe as described in the preceding paragraph, with the additional characteristic that the absorbed light of wavelength $\lambda_v$ is converted by the probe into luminescence light having wavelengths $\lambda_f$ different from $\lambda_v$. The intensity of this luminescence light, $I_f$, will then be an approximately linear function of $\alpha_v$ according to the relation

$$I_f = P_o \alpha_v \ \phi(\lambda_v/hc) \qquad \text{photons . sec}^{-1}$$

where

$\phi$ is the luminescence quantum efficiency of the probe material;
h is Planck's constant; and
c is the velocity of light in a vacuum.

If $P_o$ is kept constant, then

$$I_f = B\alpha_v \qquad \text{photons. sec}^{-1}$$

where B is a constant.

It is important to be able to measure small attenuations accurately, especially with multiplexed or distributed sensors, in which the attenuation of the interrogating light per sensing point must be kept low.

The above method is useful for increasing the accuracy of measurements from any kind of optical attenuation, not just attenuation by absorption. For example, microbending sensors work by forcing a fraction of the intensity of the interrogating light propagating along the core of an cptical fiber out of the core and into the fiber cladding. According to the teachings of this invention, one can incorporate a luminescent material or a Raman scattering material into the cladding and convert the light entering into the cladding under the action of the microbending force into either luminescence light or Raman-shifted light, the intensity of which is directly proportional to the fraction $\alpha$ removed fram the core. One can then guide the converted radiation <u>back along the same fiber</u> to the photodetection station.

Alternatively, one can carry the fraction $\alpha$ through a fiber cladding, without luminescence conversion but rendered separable from the interrogating light being propagated through the core by the time domain separation technique described in section 3.2 hereinafter. The spectral or temporal separation of the signals from the interrogating light, plus signal intensities which are orders of magnitude stronger than Rayleigh-backscatter signals, permit the multiplexing of a much greater number of sensing points on a single sensing fiber than is possible with the prior art, for the same intensity of the interrogating light and the same photodetector.

### 2.1. The Measurement of Distributed Forces with a Single Unbroken Fiber Probe.

Mechanical forces acting on an optical fiber, especially microbending forces, usually cause an attenuation of light being transmitted through the fiber core, by deflecting a fraction of the intensity of this light out of the core and into the fiber cladding. If such forces are acting on a plurality of points on a long fiber, or at a single but unknown location on it, they can usually be measured by Optical Time Domain Reflectometry (the abbreviation OTLR is used herein both for the method and for the device used for implementing it). The method consists of launching interrogating light pulses with a duration typically of the order of nanoseconds (ns) into the fiber core, and measuring the intensity of the Rayleigh-backscattered light pulses as a function of fiber distance from the fiber tip at which the interrogating light pulses were launched. Any force on the fiber which causes an attenuation of the intensity of the interrogating light pulses (for instance a microbending force) is revealed as a discontinuity in the backscatter intensity versus distance decay curve. The time of arrival of the Rayleigh-backscattered pulses, relative to the time of launching of the interrogating light pulses, defines the location along the fiber where the force is acting, and the intensity of the backscattered pulses indicates the magnitude of the force.

A serious shortcoming of this method is that it 'throws away' the deflected light to be measured, and instead estimates its magnitude indirectly from a difference between two usually much larger, but still weak and noisy backscatter signals. Rayleigh scattering is an inefficient process, producing intensities at the photodetector which are typically 50 or more dB down from the forward interrogating light intensity at any 1 meter length of fiber. These indirect measurements are, then, plagued by a relatively large amount of baseline noise.

The teachings of this invention provide devices for the measurement of force distributions on optical fibers, as well as temperature distributions. The devices of this invention are capable of producing, in the measurement of forces, signals stronger by orders of magnitude than those obtainable from Rayleigh-backscatter measurements, for the same extent of optical attenuation and with the same intensity of the interrogating light. The method consists of converting the fraction of the intensity of the interrogating light which has been deflected into the fiber cladding into a signal separable from the interrogating light transmitted through the fiber core. This separation can be achieved either in the optical wavelength domain, by luminescence or Raman conversion, or as especially claimed herein, in the time domain.

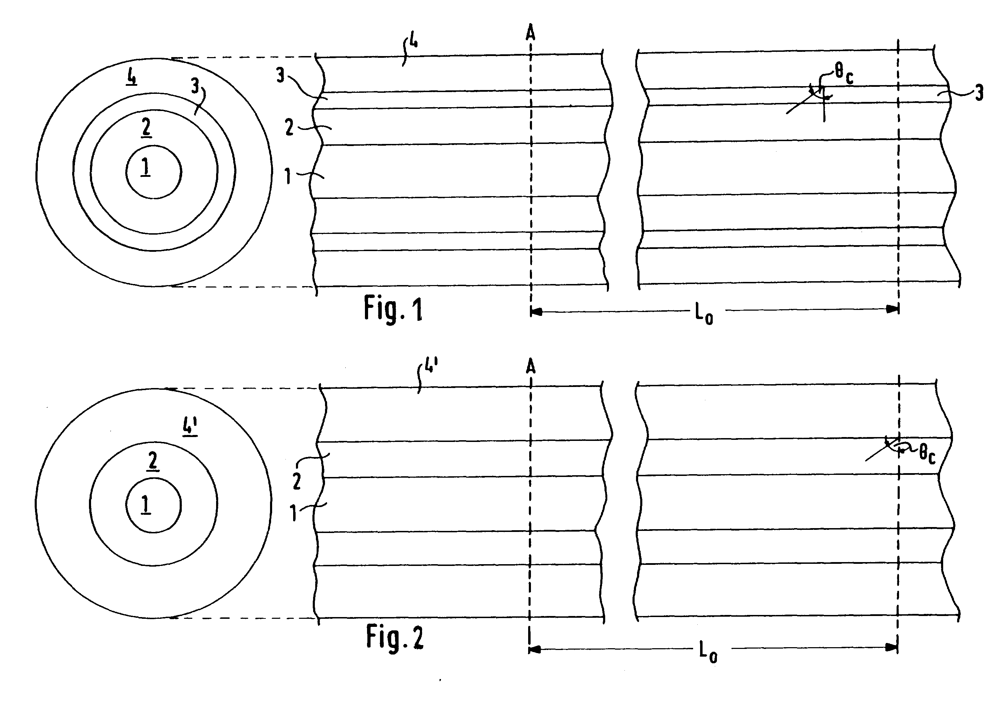

1st Example. This example uses a fiber shown schematically in FIG. 1, consisting of a glass core 1 with an index of refraction $n_1$ and a cladding 2 with an index of refraction $n_2$ tomar than $n_1$. Around cladding 2 there is a second cladding, concentric layer 3, containing fluorescent centers absent in either the core or in cladding 2 and having a luminescence decay time of the order of nanoseconds, this cladding having an index of refraction $n_3$ not lower than $n_2$ and a thickness of a few micrometers. Around cladding 3 there is a third cladding 4 with an index of refraction $n_4$ substantially lower than $n_3$. Interrogating light pulses with a duration of the order of nanoseconds, having wavelengths within the absorption band of the fluorescent centers, are launched into the core 1 of the fiber, where they propagate along the fiber axis with a small attenuation caused by residual optical absorption with a coefficient $\varepsilon$ and, usually to a larger extent, by Rayleigh light scattering with a coefficient $\beta$. At a point A along the fiber, the launched interrogating light pulses have an intensity $P_o$. After this point, and for a length L corresponding to the time resolution of the OTDR, the light intensity is attenuated by a fraction $a_t$. The power $P_s$ of the Rayleigh-backscattered light pulses generated within the fiber segment of length L within the fiber numerical aperture $(NA)_s$ and sent to the photodetection station is given approximately by

$$(P_s/P_o) \approx \tfrac{1}{2}\, a_t\, [\beta / (\beta / \varepsilon)].\, [\, (NA)_s^2 /4\, n_1^2\, ]\, f_t \text{ watts} \tag{6}$$

where the terms within the second square brackets define the fraction of the power of the scattered light captured within the solid acceptance angle of the fiber core, and $f_t$ is the fraction of the power of this captured light which is transmitted by the fiber along the optical path to the photodetector. The numerical aperture $(NA)_s$ is given by

$$(NA)_s = (\, n_1^2 - n_2^2\, )^{\tfrac{1}{2}} \tag{7}$$

An external force operating on this fiber segment and capable of producing a localized strain or any other microbending force will deflect a fraction $\alpha_s$ of the power $P_o$ of the interrogating light propagating along the fiber core at that point into cladding 2, due either to fiber microbending or to the increase of the index of refraction $n_2$ of the glass cladding relative to that of the core, the value of $\alpha_s$ being determined by the magnitude of the force. Then $P_s$ will be decreased by this fraction, and the change $\Delta P_s$ (the signal) will be given by the relation

$$\Delta P_s = \alpha_s \, P_s \text{ watts}$$

It should be noted that the optical processes described above involve only core 1 and cladding 2 and are, therefore, unaffected by layer 3 or cladding 4.

Let us now look at what happens to the light forced out of the glass core 1 and into cladding 2. For this purpose, we must treat the fiber as having a second numerical aperture $(NA)_f$, <u>for the generated fluorescence,</u> defined as

$$(NA)_f = ( n_2^2 - n_4^2 )^{\frac{1}{2}} \tag{8}$$

Light entering cladding 2 at angles within $(NA)_f$ will be trapped and propagated along the fiber axis by total internal reflection from cladding 4 but, after a number of reflections determined by the absorption coefficient and concentration of the fluorescent centers in layer 3, will be absorbed by these centers and converted into fluorescence with a quantum efficiency $\phi$. The fluorescence will usually be emitted at longer wavelengths than those of the absorbed interrogating light. The fraction $P_f$ of the power of this fluorescence light which reaches the photodetector is given approximately by

$$P_f \approx \tfrac{1}{2} \, \alpha_s \, P_o \phi \, [ \, (NA)_f^2 \, / 4 \, n_2^2 \, ] \, f_t^{'} \, (\lambda_s/\lambda_f) \text{ watts} \tag{9}$$

where

$f'_t$ is the fraction of the fluorescence power generated within $(NA)_f$ which is transmitted by the fiber along the optical path to the photodetector;

$\lambda_s$ is the wavelength of the interrogating light; and

$\lambda_f$ is the mean wavelength of the fluorescence light.

Let us now assume some typical values for some of the parameters in equations (6) and (9), for a fiber segment one meter long :

$$a_t \; 2.3 \times 10^{-3} \qquad (NA)_f = 0.40$$

$$[\beta/(\beta+\varepsilon)] = 0.80 \qquad n_1 = 1.500$$

$$\phi = 0.70 \qquad n_2 = 1.487$$

$$f_t^{'} \, /f_t = 0.70 \qquad (\lambda_s/\lambda_f) = 0.90$$

$$(NA)_s = 0.20$$

Stress or microbending forces which attenuate the intensity of the interrogating light by not more than a few percent will deflect into cladding 2 only optical rays within the numerical aperture $(NA)_f$ of the fiber, especially in view of the high value of $(NA)_f$ relative to $(NA)_s$. Thus, equation (9) applies, and one can determine from the above values of the relevant parameters that

$$P_f/\Delta P_s \approx 975$$

Fluorescence conversion of light forced out of the core of an optical fiber by stress or other forces can, thus, produce signals orders of magnitude stronger than those obtained from the decrease of the Rayleigh-backscattered light produced by the same forces. As a consequence of this, distributed sensing systems can be designed so that each sensing point attenuates a small fraction only of the intensity of the interrogating light propagating along the fiber at that point, thus allowing a given intensity of the interrogating light launched into the sensing fiber to interrogate, an produce strong signals from, a much greater number of sensing points than would be practical with a system based on Rayleigh-backscattered signals.

The above-described fluorescent claddings can be applied to maximize diagnostic information from various types of fibers, including multimode step index, graded index and single mode fibers. In the latter case, of course, only the interrogating light beam is single mode. Cladding 3 can be, for example, a clear plastic doped with an organic fluorescent dye.

<u>Second Example</u>

In the second example the light forced out of the fiber core by a stress or microbending force is converted into Raman-scattered light at a wavelength different from $\lambda_s$. This requires a different fiber from that used in the first embodiment. The fiber consists of the same or similar glass core 1 as in Figure 6. Around this core is a glass cladding having a relatively high Raman-scattering coefficient, for example a silica glass doped with a high concentration of phosphorous pentoxide, $P_2O_5$, as Raman-active material. It is important that the fiber core do or not contain the Raman-active material present in the cladding. Also, the cladding volume per unit length should preferably be greater than the core volume. Light forced out of the core and into the cladding is converted into Raman-scattered light. Generally, the Raman conversion process is less efficient than fluorescence conversion, so that the signal strengths are smaller than possible with fluorescence conversion, at least for fiber lengths of the order of a few meters.

<u>Third Example</u>

The third example is advantageous when the fluorescent material is an organic dye, and the dye can be dissolved in a transparent plastic cladding having an index of refraction substantially lower than $n_2$, the index of the glass cladding of Figure 1. This will become apparent from the following discussion.

Even the clearest plastics attenuate light by at least an order of magnitude more than the best fiber-grade glass. It is because of this fact that that the fluorescent layer 3 was specified to be only a few micrometers thick. If the diameter of cladding 2 is, for instance, 125 micrometers (a common value), then only a small fraction of the optical path of the fluorescence light is through a high loss material. But there is a better and simpler way to achieve this objective, as described below.

<u>Fluorescence Conversion by Evanescent Wave Coupling.</u>

The force-sensing fiber of this example is illustrated in Figure 2. It comprises the same core and the same cladding 2 of FIG. 1. Around cladding 2 there is a second cladding, coating 4', made of a transparent plastic and having dissolved therein a fluorescent dye characterized by a high quantum efficiency, an absorption band in a spectral region comprising the wavelength of the interrogating light, $\lambda_s$, and a fluorescence decay time of the order of $10^{-8}$ seconds or shorter. The plastic has an index of refraction $n_p$ low enough to produce a numerical aperture $(NA)_{fp}$ higher than 0.3, and preferably higher than 0.4, $(NA)_{fp}$ being defined by the relation

$$(NA)_{fp} = ( n_2^2 - n_p^2 )^{1/2} \qquad (10)$$

Light entering cladding 2 from core 1 at angles greater than the critical angle $\theta_c$ for total internal reflection from coating 4' will enter this cladding at least to a depth $d_p$ known as the thickness of the <u>evanescent layer</u>, and defined as the depth over which the electrical field amplitude of the light waves decays to the value $1/e$ of its value at the interface between cladding 2 and coating 4'. The value of $d_p$ is given by the relation

$$d_p = (\lambda_e/n_2)/2\pi \, [\sin^2\theta - (n_p/n_2)^2]^{1/2} \text{ cm} \qquad (11)$$

where $\theta$ is the angle of incidence of the light rays on the interface between cladding 2 and layer 4'(this layer can legitimately be regarded as a second cladding). Only a small fraction of the intensity of the incident light will be absorbed per reflection but, by a proper choice of the concentration of the fluorescent dye, virtually the entire intensity of the light leaving the core at angles greater than $\theta_c$ can be absorbed over the length L of the resolvable fiber segmant. The generated fluorescence can be collected by the optical fiber with high efficiency, because the angular distribution of the fluorescence will favor the modes within the acceptance angle of the fiber (Lee et. al., <u>Appl. Opt. 18,</u> 862 (1979))

### 2.2. A Practical Device for Measuring Distributed Forces According to this Invention.

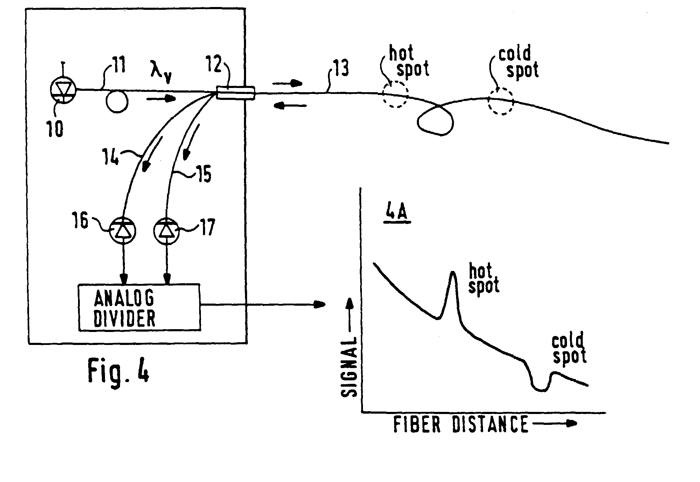

Distributed forces can be measured with the same electro-optical arrangement as that described with reference to FIG. 4, with the sensing fiber 13 being either one of the fibers illustrated in FIGS. 1 or 2. The light source 10 is driven to produce interrogating light pulses of submicrosecond duration and wavelength $\lambda_s$ within an optical absorption band of the fluorescent centers (fluorescent molecules or ions) in the applicable cladding. These light pulses are launched into the core of the sensing fiber, where they propagate along the fiber length. At any point along the fiber on which a mechanical force is acting, a fraction $\alpha$ of the intensity of the interrogating light pulses is deflected out of the core and into cladding 2, and is immediately converted into fluorescence light pulses by the fluorescent centers in the applicable cladding (cladding 3 if the fiber of FIG. 1 is used, or the evanescent layer of cladding 4' in the fiber of FIG. 2). Both the fluorescence light pulses and the Rayleigh-backscattered light pulses (from the fiber core) travel back to the electro-optical unit and, through the fiber optic coupler 12 and fiber segments 14 and 15, to photodetectors 16 and 17, which are made spectrally selective to the force-dependent fluorescence pulses and the Rayleigh-backscattered pulses, respectively. The ratio of the photo-electric signals from the two photodetectors is an indicator of the value of $\alpha$ and, hence, of the magnitude of the force. The time of arrival of the optical pulses at the photodetectors, relative to the time of launching of the interrogating light pulses, identifies the location of the force. The fluorescence light pulses are easily separable from the Rayleigh-backscattered pulses because of their different, generally longer, wavelengths.

### 2.3 The Sensing of Distributed Forces with Optical Fiber Probes Having two Light-guiding Regions of Different Effective Optical Path Length.

In the examples of force-sensing systems discussed above, I have described how light deflected out of the core of an optical fiber under the action of a force can be processed into an optical signal separable from the light transmitted by the fiber core. In the above embodiments, signal separation is effected by converting the deflected light into spectrally separable light of different wavelengths from those of the interrogating light. In this section is described distributed sensing systems in accordance with the present invention wherein signal separation is effected in the time domain, by processing the pulsed or AC-modulated light deflected out of the core into a lighthaving different temporal characteristics from those of the interrogating light carried by the fiber core, without the need for wavelength conversion. These systems do not use fibers doped with luminescent/reflective particles or any luminescent material optically linked to an optical fiber, as disclosed in DE-A-3712 699 and EP-A-0095673.

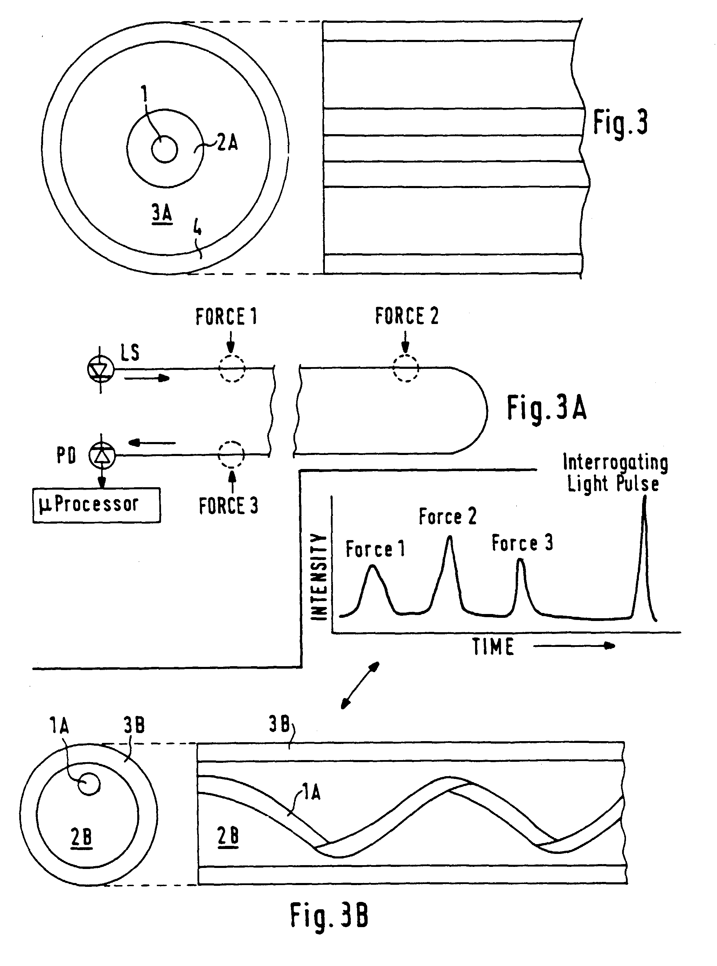

A preferred embodiment of such a technique is described in the following paragraphs, with reference to FIGS. 3 and 3A.

The optical fiber 13A shown schematically in FIG. 3 comprises a single mode core 1 with an index of refraction $n_1$, a first cladding 2A around the core having an index of refraction $n_2$ such that the value of $(n_1^2 - n_2^2)^{\frac{1}{2}}$ does not exceed 0.15, a light-guiding region 3A around cladding 2A, having a graded parabolic or near parabolic index of refraction the peak value of which, $n_3$, is substantially higher than $n_1$, and an outer cladding 4A with an index of refraction $n_4$ lower than $n_2$. The fiber is used as a distributed force-sensing probe with a device represented schematically in FIG.3A.

Referring to FIG. 3A, light source LS launches into the fiber core a train of interrogating light pulses with a duration of the order of $10^{-9}$ seconds or shorter, depending on the spatial resolution desired. At any point where a lateral force is acting on the fiber, a small fraction of the intensity of each interrogating light pulse is deflected out of the fiber core into light-guiding region 3A, where it is trapped by the graded refractive index distribution of this region and by the outer cladding 4A. Because $n_3$ is substantially higher than $n_1$, the effective optical path length of region 3A, (that is, its actual length multiplied by its index of refraction) is substantially longer than that of the core 1, so that the light deflected out of the core into region 3A arrives at the photodetector 16A at the distal fiber end after a resolvable interval $\underline{t}$ following the arrival of the interrogating light pulses carried by the fiber core. This interval identifies the location at which the deflection occurred, according to the relation

$$t = (z/c)\,(n_3 - n_1) \text{ seconds} \tag{12}$$

where

z is the fiber distance to the photodetector of the point where the the force was acting; and
c is the velocity of light in a vacuum.

For example, if $n_3$ is equal to 1.553 and $n_1$ is equal to 1.46, then two sensing points one meter apart along the fiber will produce signals arriving at the photo-detector about $3.1 \times 10^{-10}$ seconds apart, assuming that the duration of the interrogating light pulse is not longer, and that region 3A does not introduce a various pulse broadening. Since the light-guiding region 3A has a parabolic or near parabolic index profile, the broadening can be shown to be relatively low for some practical fiber lengths. The rms pulse broadening $\Delta t$ is given approximately by the relation

$$\Delta t \approx [(n_3 - n_1)/n_3]^2 [n_3 \, z/20.c.3^{\frac{1}{2}}] \text{ seconds} \qquad (13)$$

For a z distance of 100 meters and the above assumed values for $n_3$ and $n_1$, $\Delta t$ is approximately equal to $5.4 \times 10^{-11}$ seconds. The actual dispersion may be somewhat greater because of some dispersion in the first cladding 2A, but spatial resolutions of the order of 1 meter should be obtainable in fiber lengths of about 100 meters. If numerous forces are acting at different locations along the fiber, their signals will arrive at the photodetector at different resolvable times. The intensities of the time-resolved signals will be indicators of the magnitude of the forces.

Another embodiment of a fiber with two light-guiding regions of different optical path length consist of a fiber wherein one of the two light-guiding regions has an _actual_ path length different, for the same unit length of the fiber, from that of the other light-guiding region. An example of such enbodiment is a fiber as shown schematically in FIG. 3B, having a helical core 1A with an index of refraction $n_1$, a first cladding 2B around said core, having an index of refraction $n_2$ lower than $n_1$, and a second cladding 3B around the first cladding, with an index of refraction $n_3$ lower than $n_2$. When light pulses with a duration of $10^{-9}$ seconds or shorter are launched into the core of this fiber, and a lateral force deflects a fraction of the intensity of this light into cladding 2B, the cladding light pulses arrive at the photodetector _before_ the light pulses propagated by the core. The interval t' between the core pulses and the cladding pulses follows the relation

$$t' = (z/c)(n_1 C - n_2) \text{ seconds} \qquad (14)$$

where C is the ratio of the actual optical path length of the helical core to that of cladding 2B. Another embodiment uses a straight core and a helical cladding around the core.

Optical fibers having a helical light-guiding region tend to lose light at points other than those at which the forces to be measured are acting, so they are suitable for use in lengths not exceeding about 100 meters. For short fiber lengths, fibers with a helical light-guiding region have the advantage of better spatial resolution than fibers with both light-guiding regions straight, for the same duration of the interrogating light pulses.

Force sensing optical fibers having two or more light guiding regions have been described in the prior art, for example, in U S-A-4,770,492, but these prior art fibers are not suitable for (or adapted to) the sensing of distributed forces along their length or the separation in the time domain of force signals acting at different points along their length.

3.1 Fiberoptic Keyboards

The British journal Sensor Review (April 1984, pp 70-72) discusses the need for electrically passive fiberoptic keyboards in some environments where electronic keboards may be unsuitable, for example in underwater applications, in flammable atmospheres, or in electrically noisy environments, and describes a complicated experimental fiberoptic keyboard. The teachings of this invention for the trapping and modification of light deflected out of the core of an optical fiber under an acting force can be used to construct improved, simpler keyboards. In contrast to the device described in said issue of Sensor Review, which require $2.N^{\frac{1}{2}}$ sequentially pulsed light sources for a number N of keys, the fiberoptic keyboards of this invention require not more than _one_ light source. Furthermore, this single light source can be shared by numerous fiberoptic keyboards operated simultaneously.

An example of a fiberoptic keyboard using the principles of this invention uses two identical optical fibers, each comprising, a) a clear core having an index of refraction $n_1$; b) a clear cladding around said core, having an index of refraction $n_2$ lower than $n_1$ and a thickness of a few micrometers; c) a second cladding having an index of refraction $n_3$ _not_ lower than $n_2$ and a diameter preferably greater than twice the core diameter and having also a wavelength-selective light absorber dissolved therein; and d) an outer cladding with an index of refraction $n_4$ lower than $n_2$.

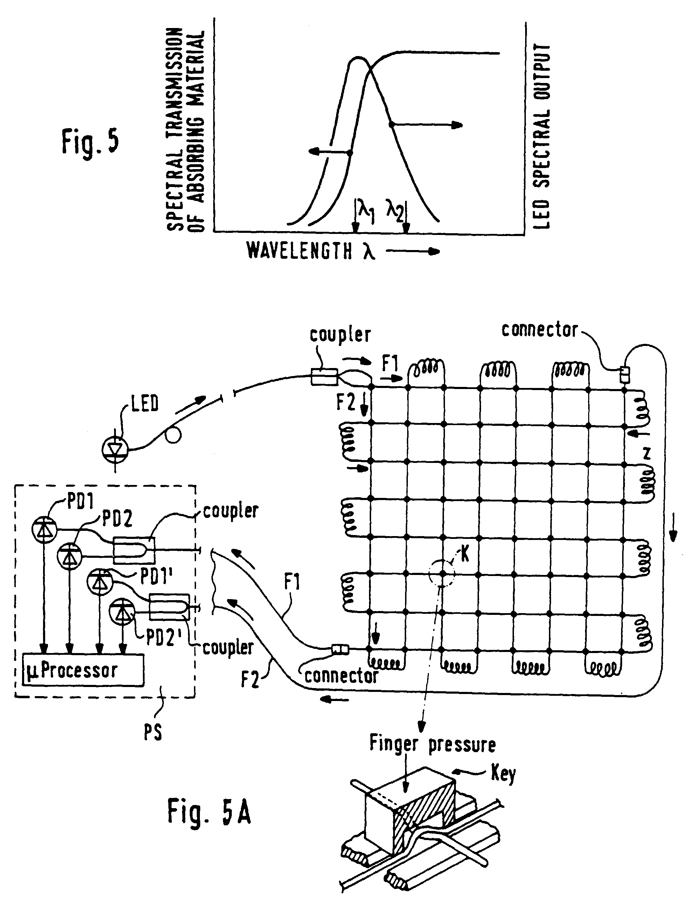

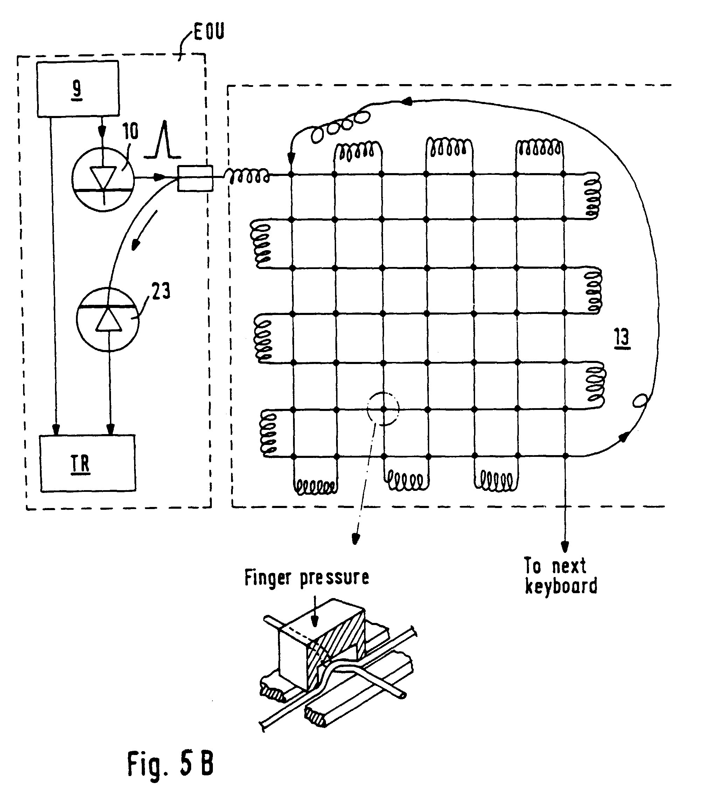

A general representation of the optical transmission spectrum of a suitable light absorber is shown in FIG. 5, which also includes a typical output spectrum of a C.W. light-emitting diode (LED) used for operating the keyboard. In the keyboard system, a length L of each fiber is used such that the total optical density to light of wavelength $\lambda_1$ propagating along the light-guiding region including the light-absorbing second cladding is about 1.3, corresponding to a transmitted light intensity P of about 5 percent of the incident light intensity $P_o$, according to the relation

$$\log(P/P_o) = - \alpha L$$

where $\alpha$ is the absorption coefficient of the light-absorbing second cladding per unit length, the product $\alpha L$ being the full-length optical density of the fiber.

FIG. 5A is a schematic representation of the operating keyboard. Two fibers, F1 and F2, are laid out as shown under an 8x8 matrix of keys, F1 passing under all rows of keys and F2 passing under all columns of keys, the two fibers intersecting under each key as shown in the area of detail under the matrix. Between each contiguos rows and columns of keys there is a fiber segment S of length z, at least 10 times longer than the fiber segment under each row or column of keys. When a key is depressed, at least an appreciable fraction of the intensity of the interrogating light launched into the core of each fiber by the LED is deflected into the light-absorbing second cladding. The deflected light propagates within the light-guiding region including said second cladding along the fiber length to the photodetection station PS, and the spectral component of wavelength $\lambda 1$ is partially absorbed. The transmitted light intensity $P_i$ of wavelength $\lambda 1$ is given by the relation

$$\log(P_i/P_o) = \alpha L(1 - N'z' - x)$$

where

z' is equal to z plus the length of fiber under a row or column of keys,
N' is the number of rows or columns (depending on whether the fiber is F1 or F2, respectively) preceding the row or column of the depressed key; and
x is the length of fiber under the preceding keys of the same row or column of the depressed key.

Since z' is more than 10 times greater than x, the value of x, determined by the number of preceding keys in the same row or column, won't affect appreciably the measured value of $P_o$. Additionally, light of wavelength $\lambda 2$ is not absorbed appreciably by the light-absorbing material. Therefore, the ratio of the light intensities of wavelengths $\lambda 1$ and $\lambda 2$ transmitted by each fiber will be a unique function of the location of the depressed key. The light outputs from each fiber are fed to two photodetector pairs, PD1-PD2 and PD1'-PD2', the digits 1 and 2 representing that the photodetectors are selectively sensitive to wavelengths $\lambda 1$ and $\lambda 2$, respectively.

For a full-length optical density of 1.30, the light intensities of wavelength $\lambda 1$ transmitted by each fiber for keys depressed at two contiguous rows or columns varies by about 30 percent for an 8x8 matrix of keys from one row or column to the next, and the percentage of variation increases as the full-length optical density increases. Therefore, the set of readings from the two sets of photodetectors, for each depressed key, will unambiguously identify the key.

The reading can be made independent of any variation of the fraction of the interrogating light intensity deflected into the absorbing cladding, by directing only the deflected light to the photodetectors. This can be achieved simply by covering the output tip of the fiber core by a black absorber, or by other means which would be apparent to workers with at least average competence in the art to which this invention pertains.

In a variant of the above method, the light-absorbing material in the second cladding is luminescent, and light of the shorter wavelengths within the luminescence spectral output is partially reabsorbed, the extent of the reabsorption varying as a function of the distance from the point under the depressed key to the distal end of the fiber, the luminescence emitted at longer wavelengths being only minimally absorbed. In this case one identifies the depressed key by the ratio of the intensities of the lights emitted at the two wavelength regions. One specific example of such a luminescent system is Nd(III)-doped glass, the luminescence spectrum of which comprises a band at about 890 nm, and another strong band at about 1060 nm (in addition to at least another band at longer wavelengths). The luminescence light within the 890 nm band is partially reabsorbed, while the band at about 1060 nm is only minimally attenuated, and can be used as a reference.

Another kind of keyboard, suitable for multiplexing, is illustrated in PIG. 5B. A light source 10, preferably an LED or a laser diode, is driven from power supply 9 to produce a recurrent train of light pulses with a duration preferably not greater than about 20 nanoseconds, and a repetition rate of $10^4$ pulses per second. At the keyboard the optical fiber 13B is laid out as shown, with intersecting horizontal and vertical segments, and with a depressable key at each inersection. Between each contiguous rows or columns of keys traversed by fiber 138 there is a fiber segment of length L of about 2.0 meters, designed to delay the time of arrival of the interrogating light pulses at any row or column by about 10 nanoseconds after the preceding row or column. At any key position, the set of fiber distances from the point of intersection to the electrooptical unit EOU is unique for that key. As the key is depressed, a small (but easily measurable) fraction of the intensity of the interrogating light progagating through the fiber core is forced into the fiber cladding and the interface between this cladding and the fluorescent coating 4'. The fluorescence light pulses generated at the evanescent wave layer of the coating arrive at the photodetector 23 at two specific times (relative to the time of injection into the fiber of the interrogating light pulses) which uniquely identify the key. The power supply 9 provides the reference timing pulse which triggers the time sweep of the timer TR. The time interval between the fluorescence pulses from contiguous rows or columns of keys is approximately 20 nanoseconds. In the illustrated example, the total length of optical fiber needed for 49 keys is 28 meters. In general, the total fiber length L needed for any number N of keys is given by

$$L = (tc/n) \cdot N^{\frac{1}{2}}$$

where

c is the velocity of light in a vacuum;
t. is the needed time resolution; and
n is the index of refraction of the glass in the fiber. (Although the indices of refraction of the core glass and the cladding glass are slightly different, they are sufficiently close to 1.50 to use this value in most design calculations).

The electro-optical unit described above can be used, without major modifications, to interrogate and read out a multiplicity of keyboards in a series array on a single fiber, or in parallel on a plurality of fibers.

The technique described above for the for the time-division-multiplexed keyboard can be used with any fiber in which light deflected from the core into another light-guiding region can be separated in the time domain from the interrogating light. The fiber 13A subject of FIG.3 is an example of an optical fiber also suitable for use in fiberoptic keyboards. In this case the light deflected under the action of a depressed key is separated in the transmission mode as discussed in section 3.2

### 3.2. The Multiplexing of Sensors on a Single Unbroken Optical Fiber.

Distributed force sensors can be used for multiplexing sensors for any physical variable which can be converted into a mechanical force. The distributed fiber sensors of this invention can receive inputs from any such sensor at any desired location, and transform those inputs into luminescence signals when the optical fiber is interrogated by a suitable light source in a device like an optical time domain reflectometer (OTDR). As explain hereinbefore, the intensities and the time characteristics of these luminescence signals define both the location along the fiber where the sensor is located and the value of the measurand. A preferred type of sensor to be coupled to the distributed sensing fibers of this invention is the well-known microbend sensor. Examples of microbend sensors which can be multiplexed on a sensing fiber of this invention are:

(a) microbenders mechanically coupled to a displacement-type sensor, for instance the diaphragm of a pressure-sensing transducer. Thus the sensor can be entirely electrically-passive; and
(b) electrically-driven microbenders, in which case only the signal transmission through the fiber will be electrically passive.

A preferred way of coupling sensor information into an optical fiber according to this invention is to convert the sensor output into an oscillatory force applied to the fiber, the oscillatory frequency being a known function of the value of the parameter being measured. A frequency signal is transmitted through an optical fiber with less degradation than an intensity signal.

### 4.0. Applications of this Invention to Fiber Optic Communications.

The provision of a luminescent waveguiding region to an optical fiber, in addition to the standard glass core and glass cladding of communication fibers, and without affecting the light propagation properties of these standard waveguiding regions, allows a plurality of new communications-related uses of the fiber, besides its use as a distributed sensing probe. Two of such uses are outlined below:

### Improved Diagnostics of Fiber Optic Networks.

In its simplest enbodiment as a distributed sensing probe, the force-sensing fiber of this invention is essentially a standard communications fiber with a standard core, a standard cladding, and a plastic coating around the cladding which, except for a few parts per million of a fluorescent dye dissolved in it, would be essentially the same as some of low index polymers presently used for the protective coating of optical fibers. Yet it is that minute concentration of the dye which enables the fiber to produce diagnostic signals from lossy points along the fiber which are orders of magnitude stronger than those from the decrease of the intensity of the Rayleigh-backscattered signals conventionally masured with OTDR diagnostic instruments, but without affecting the communication signals propagating along the fiber core. The force-sensing fiber of this invention could, therefore, be used as a communications fiber in local area networks, and its force-sensing characteristics would be a built-in diagnostic feature which would greatly extend the capability of optical time domain reflectometry. This communications fiber could be used, <u>additionally</u>, for distributed sensing, and the sensor information would be transmitted through the same fiber without interfering at all with its usual communication functions.

The Non-invasive Coupling of Information to the Fiber from the Side at Any Point.

The force-sensing fibers of this invention allow the non-invasive coupling of information into the fiber from the side at any point. The information can be coupled in digital form by any sequence and/or timing of 'force bits' applied by means of, for example, a piezoelectric transducer, when a train of short interrogating light pulses are propagating along the fiber core. The coupled information, converted either to fluorescence light pulses if the fiber has a fluorescent cladding, or to time-resolved light pulses if the fiber is like the one described in section 2.3 <u>supra</u> and FIG.3, will then propagate to at least one fiber end time and/or wavelength-separated from the interrogating light. Information can also be coupled by direct optical excitation of the fluorescent cladding by an external light source.

## Claims

1. An optical fiber having a first end and a second end and adapted to sense physical variables at a plurality of sensing points along its length, comprising at least two light-guiding regions A and B separated by a clear common cladding and so characterized that, when interrogated with light pulses of suitable short duration and of a wavelength or wavelengths within a suitable preselected spectral region injected at the first fiber end i.e. the injection end, into region A, the intensity of said light pulses propagating along the fiber at any sensing point is distributed between said regions A and B, under the action of the physical variable acting on the fiber at that point, the fiber being adapted to automatically process said relative distribution into two resolvable light pulses reaching the second fiber end, i.e., the distal end, at measurably different times determined by the location of said sensing point.

2. An optical fiber as claimed in claim 1 and adapted to sense forces, wherein said light guiding region A is a core and said light guiding region B has an effective optical path length different from that of region A, the fiber being so characterized that, when said light pulses are injected into said core A at said first fiber end, a fraction $\alpha$ of the intensity of each light pulse is deflected at any sensing point from the core to said light guiding region B, thus generating a light pulse of deflected light propagating along said region B and arriving at said second fiber end at a time measurably different from the time of arrival of the undeflected light pulse in said core and from the times of arrival of pulses of deflected light generated at other sensing points along the fiber.

3. An optical fiber as claim in claim 2 wherein said region A is the central core and is surrounded and in contact with said cladding, and said region B surrounds and is in contact with said cladding.

4. An optical fiber as claimed in claim 3 wherein said core A has an index of refraction $n_1$, said cladding has an index of refraction $n_2$, lower than $n_1$, and said light guiding region B has an effective optical path length substantially longer than that of core A.

5. An arrangement for sensing physical variables at a plurality of sensing points, comprising the optical fiber claimed in claim 1 and additionally comprising:

   a) means for generating and launching said pulses of interrogating light into the region A at the first end of said fiber; and
   b) photodetector means for receiving measuring the time of arrival, and processing said resolvable light pulses generated at each sensing point along the fiber length.

6. The sensing arrangement as in claim 5 adapted to sense forces at different locations comprising the optical fiber claimed in any of the claims 2 to 4 and wherein said

   means for generating and launching said pulses of interrogating light are adapted to inject said pulses into the fiber core A and said
   photodetector means are adapted for receiving the light pulses deflected at each sensing point to said region B.

7. The force-sensing arrangement as claimed in claim 6 and adapted to the non-invasive coupling of information into said optical fiber from the side at any point or points, the arrangement additionally comprising means for converting the information to be coupled into forces applied laterally to the fiber.

8. An optical keyboard system consisting of a force-sensing arrangement as claim in claim 7 and additionally comprising an arrangement of depressable keys, where said fiber is disposed under said arrangement of keys in such a manner that each key, when depressed, deflects a fraction of the intensity of the interrogating light injected into said core from said core into said second region simultaneously at two different points along the fiber, thus generating from each interrogating light pulse two pulses of deflected light the times of arrival of which at the second fiber end, relative to the time of arrival of the undeflected pulse, identify the location of said key.

9. The optical keyboard system as claimed in claim 8, wherein two of said optical fibers are disposed under said arrangement of keys in such a manner that the two fibers intersect under each key of said arrangement, and wherein the intensity of each interrogating light pulse is distributed between the two fibers, the depression of each key causing the deflection of a fraction of the intensity of the interrogating lights propagating along the core of each fiber at that point from said cores into said second regions, thus generating from each interrogating light pulse two pulses of deflected light the times of arrival at the distal ends of the two fibers, relative to the time of arrival of the undeflected pulses, identify the location of each key.

**Patentansprüche**

1. Lichtwellenleiter, der ein erstes Ende und ein zweites Ende hat und dafür ausgebildet ist, physikalische Variable in einer Vielzahl von Erfassungspunkten auf seiner Länge zu erfassen, und wenigstens zwei Lichtleitgebiete A und B hat, die durch einen klaren, gemeinsamen Mantel getrennt sind, und so gekennzeichnet ist, daß, wenn er mit Lichtimpulsen mit einer geeigneten kurzen Dauer und mit einer Wellenlänge oder mit Wellenlängen innerhalb eines geeigneten vorgewählten Spektralgebietes abgefragt wird, die an dem ersten Lichtewellenleiterende, d.h. dem Einleitende, in das Gebiet A eingeleitet werden, die Intensität der Lichtimpulse, die sich längs des Lichtwellenleiters ausbreiten, in jedem Erfassungspunkt zwischen den Gebieten A und B unter der Wirkung der physikalischen Variablen, die auf den Lichtwellenleiter in diesem Punkt einwirkt, verteilt wird, wobei der Lichtwellenleiter dafür ausgebildet ist, die relative Verteilung automatisch zu zwei auflösbaren Lichtimpulsen zu verarbeiten, welche das zweite Lichtwellenleiterende, d.h. das distale Ende, in meßbar unterschiedlichen Zeiten erreichen, die durch die Lage des Erfassungspunktes bestimmt werden.

2. Lichtwellenleiter nach Anspruch 1 und dafür ausgebildet, Kräfte zu erfassen, wobei das Lichtleitgebiet A ein Kern ist, wobei das Lichtleitgebiet B eine effektive optische Weglänge hat, die von der des Gebietes A verschieden ist, und wobei der Lichtwellenleiter so gekennzeichnet ist, daß, wenn die Lichtimpulse in den Kern A an dem ersten Lichtwellenleiterende eingeleitet werden, ein Bruchteil $\alpha$ der Intensität jedes Lichtimpulses von dem Kern zu dem Lichtleitgebiet B abgelenkt wird, so daß ein Lichtimpuls abgelenkten Lichtes erzeugt wird, der sich längs des Gebietes B ausbreitet und an dem zweiten Lichtwellenleiterende zu einer Zeit ankommt, die von der Ankunftszeit des nichtabgelenkten Lichtimpulses in dem Kern und von den Ankunftszeiten von Impulsen abgelenkten Lichtes, die in anderen Erfassungspunkten längs der Faser erzeugt werden, meßbar verschieden ist.

3. Lichtwellenleiter nach Anspruch 2, wobei das Gebiet A der zentrale Kern ist, von dem Mantel umgeben ist und mit diesem in Kontakt ist und wobei das Gebiet B den Mantel umgibt und mit diesem in Kontakt ist.

4. Lichtwellenleiter nach Anspruch 3, wobei der Kern A einen Brechungsindex $n_1$ hat, wobei der Mantel einen Brechungsindex $n2$ hat, der niedriger als $n_1$ ist, und wobei das Lichtleitgebiet B eine effektive optische Weglänge hat, die wesentlich länger als die des Kerns A ist.

5. Anordnung zum Erfassen von physikalischen Variablen in einer Vielzahl von Erfassungspunkten, beinhaltend den Lichtwellenleiter, der in Anspruch 1 beansprucht ist, und zusätzlich beinhaltend:

a) eine Einrichtung zum Erzeugen und Aussenden der Impulse von Abfragelicht in das Gebiet an dem ersten Ende des Lichtwellenleiters; und
b) eine Fotodetektoreinrichtung zum Empfangen, Messen der Ankunftszeit und Verarbeiten der auflösbaren Lichtimpulse, die in jedem Erfassungspunkt längs der Lichtwellenleiterlänge erzeugt werden.

**6.** Erfassungsanordnung nach Anspruch 5, die dafür ausgebildet ist, Kräfte an unterschiedlichen Orten zu erfassen, beinhaltend den Lichtwellenleiter, der in einem der Ansprüche 2 bis 4 beansprucht ist, wobei die Einrichtung zum Erzeugen und Aussenden der Impulse von Abfragelicht dafür ausgebildet ist, die Impulse in den Lichtwellenleiterkern A einzuleiten, und wobei die Fotodetektoreinrichtung dafür ausgebildet ist, die Lichtimpulse zu empfangen, die in jedem Erfassungspunkt zu dem Gebiet B abgelenkt werden.

**7.** Krafterfassungsanordnung wie im Anspruch 6 beansprucht und ausgebildet für die nichtinvasive Einkopplung von Information in den Lichtwellenleiter von der Seite her in irgendeinem Punkt oder in irgendwelchen Punkten, wobei die Anordnung zusätzlich eine Einrichtung aufweist zum Umwandeln der einzukoppelnden Information in Kräfte, die seitlich auf den Lichtwellenleiter ausgeübt werden.

**8.** Optisches Tastatursystem, das aus einer Krafterfassungsanordnung, wie sie im Anspruch 7 beansprucht ist, besteht und zusätzlich eine Anordnung von niederdrückbaren Tasten aufweist, wobei der Lichtwellenleiter unter der Anordnung von Tasten derart angeordnet ist, daß jede Taste, wenn sie niedergedrückt wird, einen Bruchteil der Intensität des Abfragelichtes, das in den Kern eingeleitet wird, aus dem Kern in das zweite Gebiet gleichzeitig in zwei verschiedenen Punkten längs des Lichtwellenleiters ablenkt, so daß aus jedem Abfragelichtimpuls zwei Impulse abgelenkten Lichtes erzeugt werden, deren Ankunftszeit an dem zweiten Lichtwellenleiterende relativ zu der Ankunftszeit des nichtabgelenkten Impulses den Ort der Taste identifiziert.

**9.** Optisches Tastatursystem nach Anspruch 8, wobei zwei Lichtwellenleiter unter der Anordnung von Tasten derart angeordnet sind, daß die beiden Lichtwellenleiter sich unter jeder Taste der Anordnung schneiden, wobei die Intensität jedes Abfragelichtimpulses zwischen den beiden Lichtwellenleitern verteilt wird und wobei das Niederdrücken jeder Taste die Ablenkung eines Bruchteils der Intensität des Abfragelichtes, das sich längs des Kerns jedes Lichtwellenleiters ausbreitet, in diesem Punkt aus den Kernen in die zweiten Gebiete bewirkt, so daß aus jedem Abfragelichtimpuls zwei Impulse abgelenkten Lichtes erzeugt werden, deren Ankunftszeit an den distalen Enden der beiden Lichtwellenleiter relativ zu der Ankunftszeit der nichtabgelenkten Impulse den Ort jeder Taste identifizieren.

**Revendications**

**1.** Fibre optique ayant une première extrémité et une seconde extrémité et adaptée de manière à détecter des grandeurs physiques variables à l'endroit d'une pluralité de points de détection répartis suivant sa longueur, comprenant au moins deux régions de guidage de la lumière A et B séparées par une gaine commune claire caractérisée en ce que, lorsqu'elle est interrogée au moyen d'impulsions de lumière d'une courte durée appropriée et d'une ou plusieurs longueurs d'onde comprises dans un domaine spectral présélectionné approprié, impulsions qui sont injectées, à l'endroit de la première extrémité de la fibre c'est-à-dire de l'extrémité d'injection, dans la région A, l'intensité de ces impulsions de lumière se propageant le long de la fibre, en un point de détection quelconque, est répartie entre les régions A et B sous l'action de la grandeur physique variable agissant sur la fibre en ce point, la fibre étant adaptée de manière à traiter automatiquement cette répartition relative pour donner deux impulsions de lumière résolubles atteignant la seconde extrémité de la fibre, c'est-à-dire son extrémité distale, à des instants différents mesurables déterminés par l'emplacement du point de détection.

**2.** Fibre optique suivant la revendication 1 et adaptée à la détection de forces, dans laquelle la région de guidage de la lumière A est un coeur et la région de guidage de la lumière B a une longueur de trajet optique effective différente de celle de la région A, caractérisée en ce que, lorsque les impulsions de lumière sont injectées dans le coeur A, à l'endroit de la première extrémité de la fibre, une fraction $\alpha$ de l'intensité de chaque impulsion de lumière est déviée, à l'endroit d'un point de détection quelconque, à partir du coeur vers la région de guidage de la lumière B, en produisant ainsi une impulsion de lumière déviée se propageant le long de la région B et arrivant à la seconde extrémité de la fibre à un instant différant, d'une manière mesurable, de l'instant d'arrivée de l'impulsion de lumière non déviée dans le coeur et des instants d'arrivée des impulsions de lumière déviées produites à l'endroit d'autres points de détection le long de la fibre.

**3.** Fibre optique suivant la revendication 2 caractérisée en ce que la région A est le coeur central et elle est entourée par la gaine et en contact avec celle-ci et la région B entoure la gaine et est en contact avec celle-ci.

**4.** Fibre optique suivant la revendication 3 caractérisée en ce que le coeur A a un indice de réfraction $n_1$, la gaine a un indice de réfraction $n_2$ inférieur à $n_1$ et la région de guidage de la lumière B a une longueur de trajet optique effective notablement plus grande que celle du coeur A.

5. Dispositif pour détecter des grandeurs physiques variables à l'endroit d'une pluralité de points de détection, comprenant la fibre optique suivant la revendication 1 et comprenant additionnellement :

   a) des moyens pour produire et lancer les impulsions de lumière d'interrogation dans la région A, à l'endroit de la première extrémité de la fibre ; et
   b) des moyens photodétecteurs pour recevoir, mesurer l'instant d'arrivée et traiter les impulsions de lumière résolubles produites à l'endroit de chaque point de détection suivant la longueur de la fibre.

6. Dispositif de détection suivant la revendication 5 adapté à la détection de forces en différents emplacements, comprenant la fibre optique suivant l'une quelconque des revendications 2 à 4 et dans lequel les moyens pour produire et lancer les impulsions de lumière d'interrogation sont adaptés de manière à injecter ces impulsions dans le coeur A de la fibre et les moyens photodétecteurs sont adaptés de manière à recevoir les impulsions de lumière déviée, à l'endroit de chaque point de détection, vers la région B.

7. Dispositif de détection de fcrces suivant la revendication 6 et adapté au couplage non invasif d'informations vers et dans la fibre optique, à partir de son côté, en un ou plusieurs points quelconques, le dispositif comprenant additionnellement un moyen pour convertir l'information devant être couplée en forces appliquées latéralement à la fibre.

8. Système de clavier optique constitué d'un dispositif de détection de forces suivant la revendication 7 et comprenant additionnellement un ensemble de touches enfonçables, dans lequel la fibre est disposée en dessous de l'ensemble des touches de telle façon que chaque touche, lorsqu'elle est enfoncée, provoque une déviation d'une fraction de l'intensité de la lumière d'interrogation, injectée dans le coeur, à partir de ce coeur vers et dans la seconde région et ce simultanément en deux points différents le long de la fibre, en produisant ainsi, à partir de chaque impulsion de lumière d'interrogation, deux impulsions de lumière déviée dont les instants d'arrivée à la seconde extrémité de la fibre, par rapport à l'instant d'arrivée de l'impulsion de lumière non déviée, identifient l'emplacement de la touche.

9. Système de clavier optique suivant la revendication 8 caractérisé en ce que deux fibres optiques sont disposées en dessous de l'ensemble des touches de telle façon que les deux fibres se croisent sous chaque touche de l'ensemble et en ce que l'intensité de chaque impulsion de lumière d'interrogation est répartie entre les deux fibres, l'enfoncement de chaque touche provoquant la déviation d'une fraction de l'intensité des lumières d'interrogation se propageant le long du coeur de chaque fibre, en ce point, à partir des coeurs et vers et dans les secondes régions, en produisant ainsi, à partir de chaque impulsion de lumière d'interrogation, deux impulsions de lumière déviée dont les instants d'arrivée aux extrémités distales des deux fibres, par rapport à l'instant d'arrivée des impulsions de lumière non déviée, identifient l'emplacement de chaque touche.

Fig. 1

Fig. 2

Fig.3

FORCE 1     FORCE 2

LS

Fig.3A

PD

μProcessor     FORCE 3

Interrogating
Light Pulse

Force 2

Force 1     Force 3

INTENSITY

TIME

1A     3B     3B

2B     1A

2B

Fig. 3B

Fig. 4

Fig. 5

SPECTRAL TRANSMISSION OF ABSORBING MATERIAL

LED SPECTRAL OUTPUT

$\lambda_1$ $\lambda_2$

WAVELENGTH $\lambda$ ⟶

coupler

connector

F1

F2

LED

PD1

PD2

coupler

PD1'

PD2'

coupler

μProcessor

PS

F1

F2

connector

Z

K

Finger pressure

Key

Fig. 5A

Finger pressure

To next
keyboard

Fig. 5 B